Europäisches Patentamt

(19) European Patent Office

Office européen des brevets

(11) Numéro de publication: **0 190 981**
**B1**

(12) ## FASCICULE DE BREVET EUROPEEN

(45) Date de publication du fascicule du brevet:
**12.04.89**

(51) Int. Cl.⁴: **A 61 F 2/32**

(21) Numéro de dépôt: **86420026.6**

(22) Date de dépôt: **30.01.86**

(54) Prothèse totale de hanche à fixation primaire.

(30) Priorité: **31.01.85 FR 8501528**

(43) Date de publication de la demande:
**13.08.86 Bulletin 86/33**

(45) Mention de la délivrance du brevet:
**12.04.89 Bulletin 89/15**

(84) Etats contractants désignés:
**BE CH DE GB IT LI NL SE**

(56) Documents cités:
**DE-A- 2 607 315**
**FR-A- 2 242 065**
**FR-A- 2 295 729**
**FR-A- 2 350 824**

(73) Titulaire: **Rhenter, Jean Luc, 57, rue Michelet,
F-42000 Saint Etienne (FR)**
Titulaire: **Collomb, Jean, L'Olagnier, F-26800 Portes Les
Valence (FR)**

(72) Inventeur: **Rhenter, Jean Luc, 57, rue Michelet,
F-42000 Saint Etienne (FR)**
Inventeur: **Collomb, Jean, L'Olagnier, F-26800 Portes
Les Valence (FR)**

(74) Mandataire: **Laurent, Michel et al, Cabinet LAURENT et
GUERRE B.P. 32, F-69131 Ecully Cédex (FR)**

ACTORUM AG

## Description

L'invention concerne une nouvelle prothèse totale de hanche à fixation primaire.

Comme on le sait, il existe essentiellement deux catégories de prothèse de hanche.

Dans la première catégorie, la prothèse est cimentée. Cette solution présente deux types d'inconvénients, à savoir:

– des inconvénients per-opératoires, dus aux chocs anesthésiques provoqués par le ciment lui-même;

– des inconvénients post-opératoires, à savoir le risque d'infection à la jonction de l'os et du ciment, des déscellements et des difficultés de reprise opératoire, s'il faut changer la prothèse ultérieurement puisque l'on a dû creuser l'os, ce qui en outre le fragilise.

La seconde catégorie, qui se développe de plus en plus, est dite «prothèse à fixation primaire». Dans cette prothèse, la tige est fixée en force dans l'os par ajustage et la repousse osseuse s'effectue sur un dessin spécial de cette tige. Ce type de prothèse est essentiellement constitué de trois pièces principales, à savoir dans l'ordre:

– une tige fémorale, destinée à être inserrée dans le fémur;

– une cupule cotyloïdienne, destinée à s'engager dans la cavité cotyloïdienne de la hanche considérée;

– un col prothétique, coiffé d'une sphère, reliant la tige et la cupule, destinée à assurer l'articulation proprement dite.

On a déjà proposé de nombreuses solutions pour réaliser de telles prothèses.

Ainsi, on a suggéré de visser la tige fémorale dans le fémur et à l'instar d'une vis, de donner à cette tige une forme légèrement tronconique, de manière à faciliter l'action de vissage (voir par exemple EP-A-0010527 et FR-A-2295729). Toutefois, cette conicité est souvent mal adaptée à la forme du fémur, ce qui entraîne un mauvais ajustage, donc une mauvaise répartition des contraintes. En outre, les pas de vis, ainsi que les profils de filets utilisés jusqu'alors occasionnent des risques de dévissage, soit par suite de la longueur insuffisante du filetage, soit par effet de rappel de la collerette d'appui. Cela entraîne des douleurs post-opératoires qui pourraient être provoquées par des pics de contrainte au niveau du filet.

Dans le brevet français FR-A-2242065, on a proposé une prothèse de hanche dont la tige fémorale présente des nervures porteuses circulaires arrondies et qui correspondent par leur forme au profil fileté d'un tire-fond. La section longitudinale de cette tige est évasée depuis la pointe jusqu'à la tête, mais de manière assymétrique. En outre, le rayon de courbure de la courbe enveloppe diminue par paliers depuis la pointe jusqu'à la tête. De plus, cette section longitudinale présente deux rayons distincts non symétriques, l'un extérieur, l'autre intérieur. C'est la raison pour laquelle cette tige doit être emmanchée par force, son ancrage s'effectuant sans ciment grâce auxdites nervures.

En revanche, l'extraction de cette tige est très délicate, ce qui n'autorise guère une réparation ou une nouvelle intervention.

L'invention pallie ces inconvénients. Elle vise une prothèse totale de hanche à fixation primaire qui ne présente pas les inconvénients énumérés ci-dessus.

En d'autres termes, l'invention vise une prothèse totale de hanche filetée à fixation primaire qui respectivement:

– soit mieux adaptée à la forme du fémur et par voie de conséquence, soit mieux ajustée;

– diminue les pics de contrainte au niveau du filet;

– améliore la fixation primaire et diminue la résection de l'extrémité supérieure du fémur;

– permette une orientation idéale de l'antéversion du col prothétique;

– et enfin, soit facile à visser ou à dévisser.

Cette prothèse totale de hanche à fixation primaire, formée de trois parties principales:

– une cupule cotyloïdienne, destinée à s'engager dans la cavité cotyloïdienne de la hanche considérée;

– un col prothétique coiffé d'une sphère reliant la tête de la tige et la cupule, destiné à assurer l'articulation proprement dite;

– une tige fémorale, destinée à être insérée dans le fémur, évasée depuis la pointe jusqu'à la tête, dont la section est circulaire et dont le rayon de courbure de la courbe enveloppe diminue depuis la pointe jusqu'à la tête;

caractérisée:

– en ce que ladite tige fémorale est filetée et est symétrique par rapport à son axe longitudinal;

– et en ce que la diminution du rayon de courbure de la courbe enveloppe se fait en permanence de manière progressive et continue, depuis la pointe jusqu'à la tête.

En d'autres termes, l'invention concerne une prothèse totale de hanche à fixation primaire dont la tige filetée fémorale se caractérise par un profil anatomique particulier en forme de tulipe à rayon progressivement variable.

Par «profil à rayon progressivement variable», il faut entendre un profil longitudinal dont le rayon de courbure varie en permanence de manière progressive et continue depuis la pointe de la tige, où il est alors à son maximum, jusqu'à sa tête, et par voie de conséquence dont le rayon de la section croît progressivement de la pointe à la tête.

Avantageusement, en pratique:

– le pas de vis de la tige fémorale fileté, de même que le pas de vis de la cupule, présentent une section trapézoïdale à pans coupés, dans lequel avantageusement les raccords de ces pans coupés sont arrondis;

– dans ce pas de vis trapézoïdal, la face supérieure, par rapport à la direction du pas de vis, est moins inclinée que la face inférieure par rapport au même plan de la section transversale de la tige;

– l'angle d'inclinaison de la face supérieure par rapport au plan de la section transversale est compris entre 15 et 25°, de préférence voisin de 20°, et l'angle d'inclinaison de la face supérieure par rapport à ce même plan, est compris entre 10 et 20°, de préférence voisin de 15°;

– la tige filetée présente également des fentes longitudinales disposées selon des génératrices, dont les angles d'attaque sont inclinés notamment dans les deux directions, pour faciliter l'opération de vissage et de dévissage;

– l'emboitement du col prothétique sur la tête de la tige filetée et sur la tête d'articulation prothétique en forme de sphère est réalisé au moyen de deux alésages coniques à faible pente, respectivement mâle et femelle.

La manière dont l'invention peut être réalisée et les avantages qui en découlent ressortiront mieux de l'exemple de réalisation qui suit donné à titre indicatif et non limitatif, à l'appui des figures annexées.

La fig. 1 montre une vue en coupe d'une prothèse totale à fixation primaire conforme à l'invention;

la fig. 2 est une représentation d'une section selon l'axe I–I' de la fig. 1;

la fig. 3 montre en détail la tige filetée caractéristique de l'invention de la fig. 1, alors que la fig. 4 est une vue en section transversale selon l'axe II–II' de la fig. 3;

la fig. 5 est une vue agrandie du pas de vis caractéristique de l'invention et plus précisément de la partie encerclée référencée V montrée à la fig. 3.

En se référant aux figures, la prothèse totale selon l'invention se compose tout d'abord d'une tige fémorale filetée de section circulaire (1), dont la tête (2) est plus large que la pointe (3) et qui, selon la caractéristique de l'invention, présente une section longitudinale (4) en forme de tulipe à rayon variable, évasée depuis la pointe (3) jusque vers la tête (2). Ce profil caractéristique évasé anatomique, présente une courbure qui varie en permanence de manière progressive et continue, donc à rayon variable, dont le profil répond à un polynome de degré trois. A titre indicatif, dans une forme de réalisation avantageuse, le diamètre de la tige (1) à la pointe (3) est de dix-huit millimètres (pas de vis compris), à l'extrémité opposée vers (2) de trente millimètres, alors qu'en son milieu il est seulement de vingt millimètres et demi, pour une longueur de filetage totale de quatre vingt quinze millimètres.

Cette tige (1) peut être réalisée en tout matériau usuel, tel que par exemple acier inoxydable, alliage métallique, en céramique. Avantageusement, cette tige (1) est en titane et de préférence la surface extérieure de cette tige est recouverte d'une fine couche de céramique, notamment d'oxyde d'aluminium.

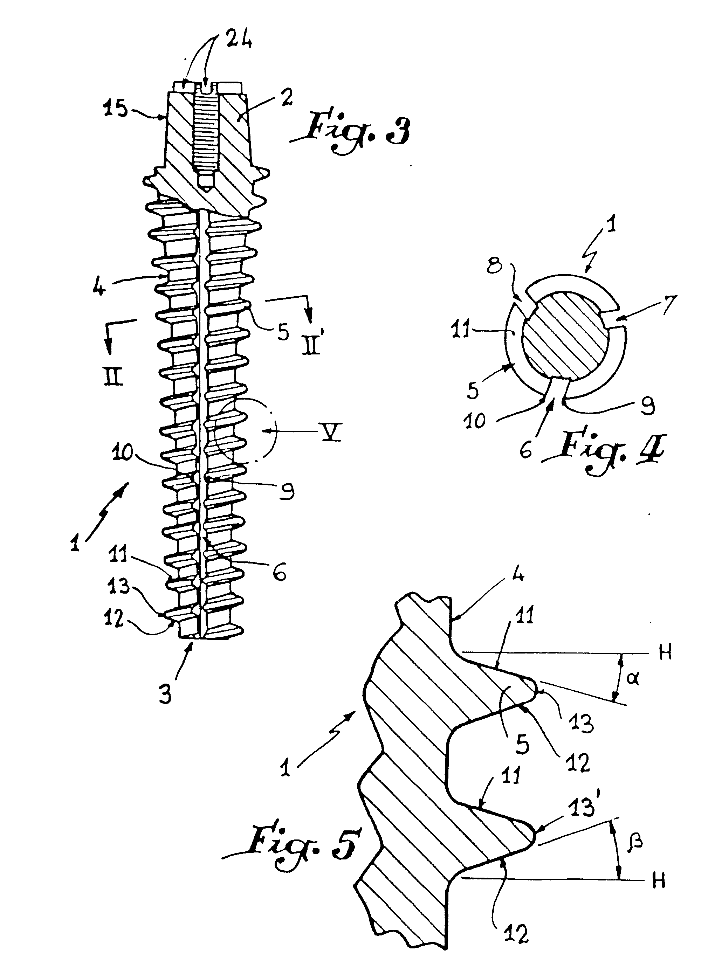

Cette tige (1) comporte un filet (5) dont le pas de vis présente une section trapézoïdale à pans coupés arrondis, montré en détail aux fig. 3 et 5.

La tige (1) comporte également sur ses génératrices trois fentes longitudinales respectivement (6, 7 et 8), décalées angulairement de 120°, dont les angles d'attaque (9) et (10) sont inclinés notamment dans les deux directions pour permettre le vissage et le dévissage. En pratique, ces angles d'attaque ont des angles vifs. Ainsi, ces trois fentes (6, 7, 8) qui forment des encoches débouchantes, facilitent l'action de vissage et s'il y a lieu de dévissage par autotaraudage et permettent avantageusement de diminuer la quantité de matériel ancillaire.

Selon une autre caractéristique originale de l'invention (voir fig. 3 et 5), le pas de vis (5) de la tige (1) comporte une section trapézoïdale à pans coupés arrondis. Dans ce pas de vis (5), la face supérieure (11) du trapèze par rapport à la direction du pas de vis, c'est-à-dire la direction de vissage, est moins inclinée que la face inférieure (12) de ce même trapèze. L'angle $\alpha$ formé entre la face supérieure (11) et l'horizontale H est compris entre 10 et 20°, de préférence voisin de 15°, afin de participer à l'angle d'attaque et d'assurer une bonne tenue de la tige (1) dans le fémur. L'angle $\beta$ formé entre cette même horizontale H et la face inférieure (12) est compris entre 15 et 25°, et de préférence voisin de 20°, afin de ménager un angle d'attaque efficace. Comme on le voit à la fig. 5, les raccords (13, 13') entre les pans coupés (11, 12) sont arrondis.

De manière inattendue, le profil caractéristique tulipé à rayon variable s'adapte de façon presque parfaite à la forme des corticales de l'extrémité supérieure du fémur. De ce fait, ce profil permet un excellent ajustage de la tige et améliore la répartition des contraintes entre la prothèse et l'os.

De même, de manière inattendue, le pas de vis trapézoïdal connu par ailleurs, présente dans l'application de la prothèse de hanche des avantages spécifiques, car non seulement il améliore la résistance à l'arrachement, mais surtout la répartition des contraintes et par là, diminue les pics de contrainte.

La tête (2) proprement dite de la tige (1) se termine par une portée conique (15) dont l'angle au sommet est voisin de 6°. Cette portée conique mâle (15) vient s'emboiter dans la partie femelle correspondante de la base (16) du col prothétique (17) et ce, dans un cône femelle (18) de forme correspondante.

Le col prothétique (17) réalisé dans le même matériau que la tige (1), par exemple en titane, présente donc à sa base (16) une partie légèrement cônique femelle (18), et à sa tête (19) une autre portée conique supérieure (20) destinée à venir s'emboiter dans une portée femelle conique (25) correspondante de la tête prothétique (21) proprement dite en forme de sphère. L'angle du cone d'emboitage (19, 20) est voisin de l'angle du cone d'emboitage (15, 18), c'est-à-dire est voisin de trois degrés (cone de morse). Cet emboitement, bien connu en mécanique, et déjà proposé en prothèse (voir par exemple brevets FR-A-1017927 et EP-A-0000549) est facile à réaliser. En outre, il permet une orientation idéale de l'antéversion du col prothétique fémoral (17).

L'assemblage du col prothétique (17) sur la tige (1) a été effectué au moyen d'une vis (23) de copatation.

L'angle formé entre d'axe longitudinal du col prothétique (17) et l'axe longitudinal de la tige (1) est voisin de 135°.

La tête prothétique (21) en forme de sphère, vient ensuite s'emboiter sur la cupule cotyloïdienne qui, de manière connu, est destinée à venir s'engager dans la cavité cotyloïdienne de la hanche considérée.

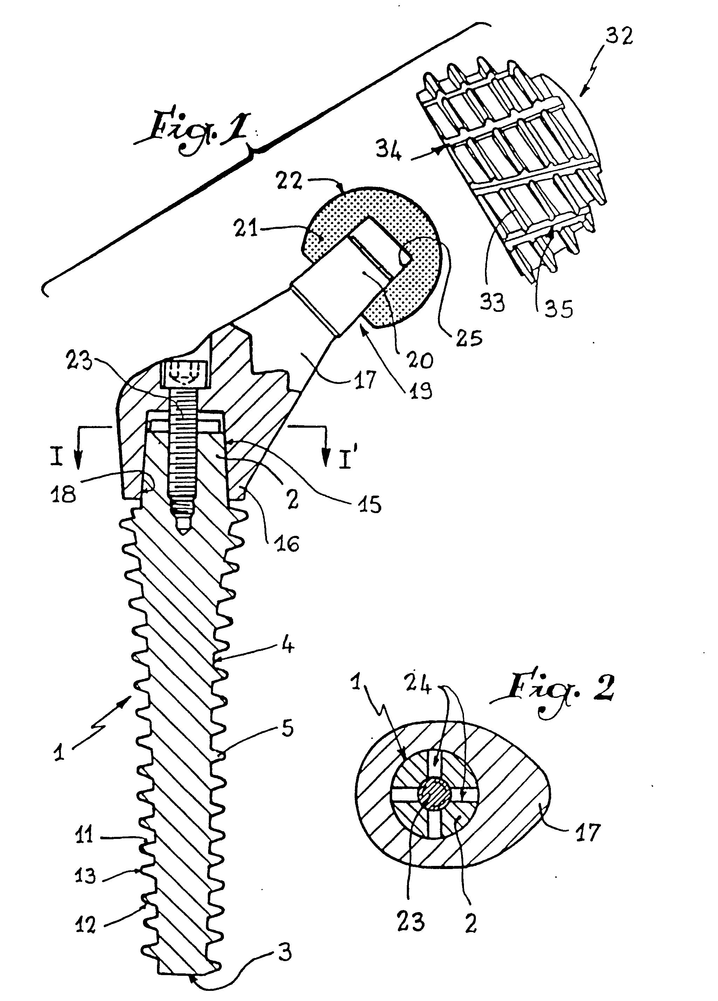

Cette cupule (voir fig. 1) se compose tout d'abord d'une pièce classique non représentée, en polyéthylène haute densité, dont la forme interne creuse est destinée à venir s'appuyer sur la partie frottante (22) de la tête (21) et dont la partie externe vient s'encastrer dans la cupule proprement dite (32) qui forme ainsi réceptacle et présente une forme extérieure ovoïde. Dans une forme avantageuse, cette partie frottante (22) est constituée par une couche de nitrure de titane déposée en phase gazeuse, par exemple sur une épaisseur de trois à dix microns. La cupule (32) également en titane a une forme extérieure qui correspond sensiblement à celle du cotyle, ce qui permet d'améliorer la transmission des forces au bassin. Cette cupule (32) présente également un pas de vis trapézoïdal (33) analogue au pas de vis (5) et également une série d'encoches longitudinales (34, 35) autotaraudeuses.

Avantageusement, le pas de vis trapézoïdal (33) de la cupule (32) à pans coupés arrondis présente les mêmes caractéristiques que le pas de vis (5) de la tige (1). De même, dans une forme d'exécution avantageuse, le pas de vis de la tige (1) et celui de la cupule (32) est de six.

Les prothèses selon l'invention présentent de nombreux avantages par rapport aux solutions commercialisées à ce jour. On peut citer:

– la diminution, voire la disparition des pics de contrainte au niveau du pas de vis résultant de manière tout à fait inattendu du choix de la forme particulière de ce pas de vis, à savoir la section trapézoïdale à pans coupés arrondis, diminution des pics de contrainte qui doit diminuer notablement les douleurs post-opératoires;

– la forme de tulipe légèrement évasée à rayon variable qui est mieux adaptée à la forme du fémur que la forme conique et par voie de conséquence, permet un meilleur ajustage anatomique;

– l'absence de collerettes qui diminue l'importance de la resection osseuse de l'extrémité supérieure du fémur et par voie de conséquence le risque de fracture du trochanter;

– enfin, grâce à l'emboitement facile par cône à faible pente, bien connu en mécanique et pour ce type d'application, une orientation idéale de l'antéversion du col prothétique pour chaque cas, ce que l'on ne savait obtenir jusqu'alors;

– enfin la simplicité de pose avec peu de matériel ancillaire du fait du caractère auto-taraudant de la tige.

## Revendications

1. Prothèse totale de hanche à fixation primaire, constituée de trois parties principales:
– une cupule cotyloïdienne (32), destinée à s'engager dans la cavité cotyloïdienne de la hanche considérée;
– un col prothétique (17) coiffé d'une sphère (21) reliant la tête (15) de la tige (1) et la cupule (32), destiné à assurer l'articulation proprement dite;
– une tige fémorale (1), destinée à être insérée dans le fémur, évasée depuis la pointe (3) jusqu'à la tête (2), dont la section est circulaire et dont le rayon de courbure de la courbe enveloppe diminue depuis la pointe (3) jusqu'à la tête (2), caractérisée:
– en ce que ladite tige fémorale est filetée (5) et est symétrique par rapport à son axe longitudinal;
– et en ce que la diminution du rayon de courbure de la courbe enveloppe se fait en permanence de manière progressive et continue, depuis la pointe (3) jusqu'à la tête (2).

2. Prothèse selon la revendication 1, caractérisée en ce que le pas de vis (5) de la tige (1) présente une section trapézoïdale à pans coupés arrondis.

3. Prothèse selon la revendication 1, caractérisée en ce que dans le pas de vis trapézoïdal (5, 33), l'angle d'inclinaison de la face supérieure (11) par rapport au plan de la section transversale est compris entre 15 et 25°, et de préférence de 20°, et en ce que l'angle d'inclinaison de la face inférieure (12) par rapport à ce même plan est compris entre 10 et 20°, de préférence voisin de 15°.

4. Prothèse selon l'une des revendications 1 à 3, caractérisée en ce que la tige (1) comporte des fentes (6, 7, 8) longitudinales, disposées selon des génératrices dont les angles d'attaque (9, 10) sont inclinés, dans les vissage et dévissage.

5. Prothèse selon l'une des revendications 1 à 4, caractérisée en ce que l'emboitement du col prothétique (17) respectivement sur la tête (15) de la tige filetée (1) et sur la tête (19) d'articulation prothétique coiffée par la sphère (21), est réalisé au moyen de deux alésages coniques à faible pente, respectivement mâle (19), femelle (18).

6. Prothèse selon l'une des revendications 1 à 5, caractérisé en ce que la tige (1), le col prothétique (17), la sphère (21) et la cupule (32) sont en titane.

7. Prothèse selon la revendication 6, caractérisée en ce que la partie frottante (22) de la tête (21) en forme de sphère est revêtue d'une couche de nitrure de titane qui coopère avec une pièce en polyéthylène haute densité en forme de creux, encastrée dans la cupule (32).

## Patentansprüche

1. Total-Endoprothese für das Hüftgelenk zur primären Fixierung, die drei Hauptteile aufweist, nämlich:
– eine hüftgelenkpfannenartige Kuppel (32), die dazu vorgesehen ist, dass sie in die Gelenkpfanne für den Oberschenkel der betreffenden Hüfte eingreift;

– einen, mit einer Kugel (21) bedeckten Prothesenhals (17), der den Kopf (15) eines Schaftes (1) und die Kuppel (32) miteinander verbindet und der dazu vorgesehen ist, dass er für die eigentliche Gelenkverbindung sorgt;

– einen, zum Einsetzen in den Oberschenkelknochen vorgesehenen Oberschenkelschaft (1), der sich von einer Stelle (3) zum Kopf (2) hin erweitert, dessen Querschnitt kreisförmig ist und bei dem sich der Krümmungsradius der Mantelkurve von der Stelle (3) bis zum Kopf (2) hin verringert, dadurch gekennzeichnet, dass

– der Oberschenkelschaft mit einem Gewinde (5) versehen und im Hinblick auf seine Längsachse symmetrisch ist; und

– dass die Verringerung des Krümmungsradius der Mantelkurve zwischen der Stelle (3) bis zum Kopf (2) hin andauernd und zwar zunehmend und stetig ist.

2. Prothese nach Anspruch 1, dadurch gekennzeichnet, dass der Gewindegang (5) des Schaftes (1) im Schnitt trapezörmig mit abgeschrägten, abgerundeten Kanten ist.

3. Prothese nach Anspruch 2, dadurch gekennzeichnet, dass beim trapezförmigen Gewindegang (5, 33) der Neigungswinkel der oberen Fläche (11) im Hinblick auf eine Querschnittsebene zwischen 15 und 25°, vorzugsweise 20° beträgt, und dass der Neigungswinkel der unteren Fläche (12) im Hinblick auf dieselbe Ebene zwischen 10 und 20°, vorzugsweise in der Nähe von 15° liegt.

4. Prothese nach einem der Ansprüche 1 bis 3, dadurch gekennzeichnet, dass der Schaft (1) längsverlaufende, entlang von Mantellinien angeordnete Einschnitte (6, 7, 8) aufweist, deren Eingriffswinkel (9, 10), in Ein- und Ausschraubrichtung gesehen, geneigt sind.

5. Prothese nach einem der Ansprüche 1 bis 4, dadurch gekennzeichnet, dass die Steckanschlüsse des Prothesenhalses (17) am Kopf (15) des Gewindeschafts bzw. am Prothesengelenkkopf (19), der durch die Kugel (21) bedeckt ist, durch eine steckerartige (19) bzw. eine buchsenartige (18) konische Bohrung mit geringer Neigung gebildet werden.

6. Prothese nach einem der Ansprüche 1 bis 5, dadurch gekennzeichnet, dass der Schaft (1), der Prothesenhals (17), die Kugel (21) und die Kuppel (32) aus Titan sind.

7. Prothese nach Anspruch 6, dadurch gekennzeichnet, dass der gleitende Bereich (22) des Kugelkopfes (21) mit einer Schicht an Titannitrid überzogen ist, die mit einem Element aus Polyethylen hoher Dichte in Form eines Kreuzes zusammenwirkt, das in die Kuppel (32) eingebaut ist.

**Claims**

1. A total hip prosthesis with primary fixation, comprising three main parts:
   – a cotyloidal cupule (32) designed to be engaged in a cotyloidal cavity of the treated hip;
   – a prosthetic neck (17), topped with a sphere (21) joining the head (15) of the pin (1) with the cupule (32), designed to ensure the articulation proper;
   – a femoral pin (1), designed to be inserted into the femur, widered from the point (3) to the head (2), the cross-section of which being circular and the curvature enveloping radius of which being decreasing from the point (3) until the head (2); characterized by the facts that:
   – the said femoral pin is screwed (5) and is symetrical with respect to its longitudinal axis;
   – the decrease of the curvature radius of the enveloping curve is permanent, continuous and progressive, from the point (63) to the head (2).

2. Prosthesis according to claim 1, wherein the thread (5) of the pin (1) has a bevelled trapezoïdal cross-section with rounded edges.

3. Prosthesis according to claim 2, wherein into the trapezoïdal thread (5, 33), the inclination angle of the upper face (11) with respect to the plane of the transverse section is comprised between 15 and 25°, and wherein the inclination angle of the lower face (12) with respect to the said place is between 10 and 20°, preferably in the vicinity of 15°.

4. Prosthesis according to any claims 1 to 3, wherein the pin (1) comprises longitudinal slots (6, 7, 8), provided on its generatrices, the angles of incidence (9, 10) of which being inclined during screwing and unscrewing.

5. Prosthesis according to any claims 1 to 4, wherein the fitting of the prosthetic neck (17) respectively on the head (15) of the screwed pin (1) and on the prosthetic articulation head (19) topped with the sphere (21), is achieved by means of two slightly conical borings, respectively male (19) and female (18).

6. Prosthesis according to any claims 1 to 5, wherein the pin (1), the prosthetic neck (17), the sphere (21) and the cupule (32) are in titanium.

7. Prosthesis according to claim 6, wherein the rubbing part (22) of the spherical head (21) is coated with a layer of titanium nitride which cooperates with a hollow high density polyethylen piece, embedded into the cupule (32).

Fig.1

Fig. 2

*Fig. 3*

*Fig. 4*

*Fig. 5*

9